# EUROPEAN PATENT APPLICATION

(11) **EP 4 289 267 A1**
(43) Date of publication of application: **13.12.2023**
(21) Application number: 22748752.7
(22) Date of filing: 04.02.2022
(51) Int. Cl.: A01N 3/02, C07K 7/06, C07K 7/08, A23L 3/3526

(54) **ADDITIVE FOR PRESERVING CELLS, TISSUE AND/OR FOODSTUFFS, AND USE**

(30) Priority: 08.02.2021 BR 102021002393
(71) Applicant: Proteimax Bio Technology Israel Ltd, Caessarea 3088900 (IL)
(72) Inventor: HEIMANN, Andrea Sterman, São Paulo (BR)
(74) Representative: Pace Napoleone, Maria
(86) International application number: PCT/BR2022/050037
(87) International publication number: WO 2022/165578

(57) **Abstract**

The present invention lies in the fields of Biotechnology, Phytochemistry and Food Technology and provides an additive for preservation of cell, tissue and/or food and the use of a synthetic peptide for preparing said additive. The invention is useful for, among other applications, increasing the shelf life of foods and providing greater food safety. In one embodiment, the additive of the invention provides substantial fruit preservation upon a single application of the additive, providing for the treatment of plants or food to prevent the occurrence of pests such as bacteria or fungi, such as, for example, those related to fruit rot, including strawberries, *Xylella fastidiosa* in coffee and orange trees, In one embodiment, the additive of the invention provides substantial preservation of flowers or inflorescences upon a single application of the additive, providing an alternative against rapid wilting, rot or fungal growth associated with the storage, transport and/or medicinal processing of flowers or inflorescences of phytochemical interest, including *Gerbera, Cannabis,* among others. In one embodiment, the present additive provides substantial preservation of the metabolic state of sprouts or seedlings, as well as slows down the seed germination process when said additive is applied, thus being useful for synchronizing germination, adjusting time of planting or germination, among other agronomic or botanical advantages.

## Description

### Field of the Invention

The present invention lies in the fields of Biotechnology, Phytochemistry and Food Technology and provides an additive for preservation of cell, tissue and/or food and the use of a synthetic peptide for preparing said additive. The invention is useful for, among other applications, increasing the shelf life of foods and providing greater food safety. In one embodiment, the additive of the invention provides substantial fruit preservation upon a single application of the additive, providing for the treatment of plants or food to prevent the occurrence of pests such as bacteria or fungi, such as, for example, those related to fruit rot, including strawberries, *Xylella fastidiosa* in coffee and orange trees. In one embodiment, the additive of the invention provides substantial preservation of flowers or inflorescences upon a single application of the additive, providing an alternative against rapid wilting, rotting or fungal growth associated with the storage, transport and/or medicinal processing of flowers or inflorescences of phytochemical interest, including *Gerbera, Cannabis,* among others. In one embodiment, the present additive provides substantial preservation of the metabolic state of sprouts or seedlings, as well as slows down the seed germination process when said additive is applied, thus being useful for synchronizing germination, adjusting time of planting or germination, among other agronomic or botanical advantages.

### Background of the Invention

The phytochemical and food markets require constant evolution to meet production, logistics and consumption needs. In this context, solutions that benefit any of these points in the chain are highly desirable. The present invention provides a solution that applies to all these elements of the respective chains.

In the search for the state of the art in scientific and patent literature, the following documents were found that deal with the subject:
Patent application BR 102014000521-0, by the present inventor, is considered the closest antecedent to the present invention. However, neither this nor any of the other documents found reveal, suggest, or teach the topical use of a peptide for preservation of cells and/or food and an additive comprising said synthetic peptide.

From what can be seen from the researched literature, no documents were found anticipating or suggesting the teachings of the present invention, so that the solution proposed here has, in the eyes of the inventor, novelty and inventive activity compared to the state of the art.

### Summary of the Invention

The present invention solves problems of the state of the art by the use of a synthetic peptide for preservation of cells, tissues and/or food and an additive comprising said peptide, being useful to provide improvements in the production, logistics and/or consumption of food and/or plants.

It is one of the objects of the invention to provide an approach to increase the shelf life of foods and provide greater food safety.

It is another object of the invention to provide an approach to increase the quality and/or shelf life of plants or parts thereof to provide improvements in the production chain of phytochemicals.

In one embodiment, the invention provides a topical additive comprising said peptide, for preservation of cells, tissues and/or food and water or an excipient.

In one embodiment, the invention provides an additive for use in the hydration liquid of the stem or branch of a flower or inflorescence of ornamental or phytochemical interest.

The synthetic peptide in the present invention is selected from the peptide of SEQ ID NO: 1 and SEQ ID NOs: 4 to 14, optionally additionally containing a cell-penetrating peptide tail at the N-terminal portion and/or at the C-terminal portion of the first and/or the last amino acid. The peptide of SEQ ID NO: 3 represents a peptide containing the peptide of SEQ ID NO: 1 and the cell-penetrating peptide of SEQ ID NO: 2 in the C-terminal region. The peptide of SEQ ID NO: 15 represents a peptide containing the peptide of SEQ ID NO: 9 and the cell-penetrating peptide of SEQ ID NO: 2.

In one embodiment, the present additive provides substantial fruit preservation upon application of at least one dose.

In one embodiment, the present additive provides for the treatment of plants or food to prevent the occurrence of pests such as bacteria or fungi, such as, for example, those related to the rot or contamination of strawberries.

In one embodiment, the present additive provides substantial preservation of the metabolic state of sprouts or seedlings, as well as slows down the seed germination process when said additive is applied, thus being useful for synchronizing germination, adjusting time of planting or germination, among other agronomic or botanical advantages.

In one embodiment, the present additive provides for the treatment of flowers or inflorescences of ornamental or phytochemical interest, to prevent or delay wilting, rot or the occurrence of pests such as bacteria or fungi, and maintain the quality or integrity of the flowers or inflorescences for a longer period.

These and other objects of the invention will be immediately valued by those versed in the art and by companies with interests in the segment, and will be described in sufficient detail for their reproduction in the following description.

### Description of the Drawings

In order to better define and clarify the content of the present patent application, the present figures are presented:

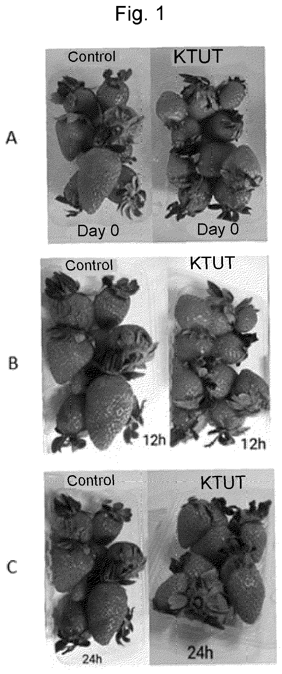

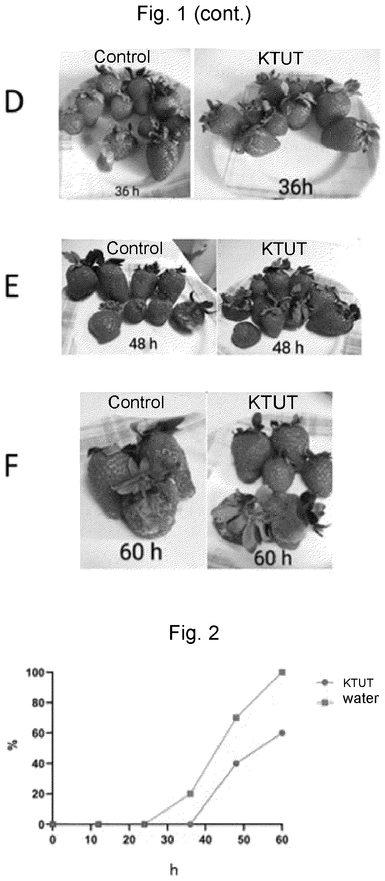

Fig. 1 shows the visual results of an experiment applying 100 µM of the preservative peptide in the Ktut embodiment, SEQ ID NO: 3 or water (control) on fresh strawberries, all from the same batch and the same commercial product packaging. The results of groups of 10 strawberries in each condition (test and control) at 0, 12, 24, 36, 48 and 60h, respectively in A, B, C, D, E and F are shown.
Fig. 2 shows the time in hours (abscissa) and the percentage of spoiled fruits (ordered) after application of 100 µM of an embodiment of the preservative peptide (Ktut, SEQ ID NO: 3) or water on fresh strawberries.

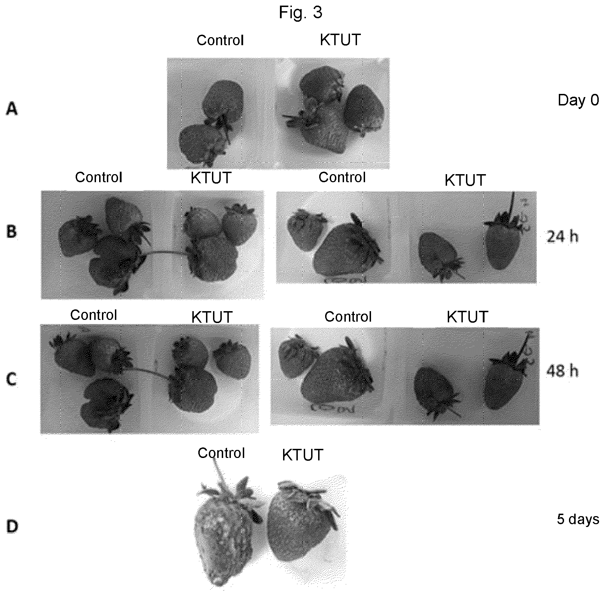

Fig. 3 shows the visual results of an experiment applying additives of the invention, wherein 100 µM of an embodiment of the preservative peptide (Ktut, SEQ ID NO: 3) in water, or only water as a control on 8 fresh strawberries in each group, all from the same batch and packaging as the commercial product. Representative results of strawberries in each condition (test and control) at 0h, 24h, 48h and 5 days, respectively in A, B, C and D are shown.

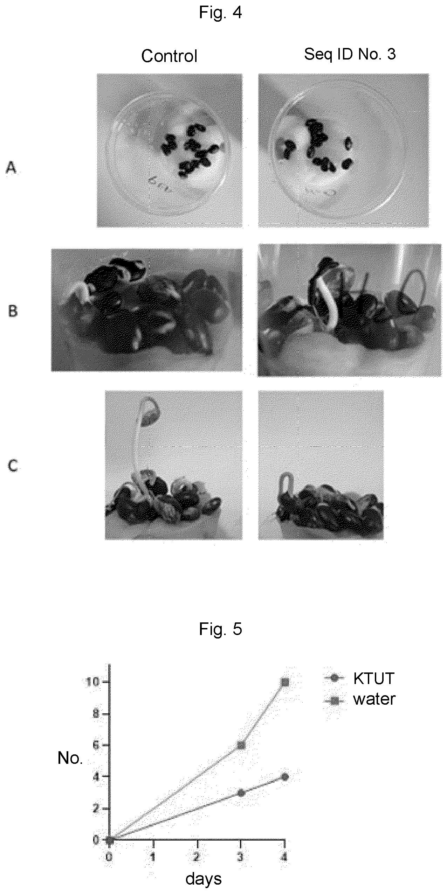

Fig. 4 shows the visual results of an experiment applying a single dose of 100 µM (in water) of an embodiment of the preservative peptide (Ktut, SEQ ID NO: 3) or water (control) on bean seeds, all from the same batch and same packaging as a commercial product and kept at room temperature (28 °C). The results of germination of groups of 14 seeds in moist cotton in each condition (control and test) in 0, 3 and 4 days, respectively in A, B and C are shown. The images show that there were differences in the germination pattern of the beans.
Fig. 5 shows the time in days (abscissa) and the number of bean seeds that germinated (ordered) after application of 100 µM of an embodiment of the preservative peptide (Ktut, SEQ ID NO: 3) under the conditions indicated in Fig. 4.

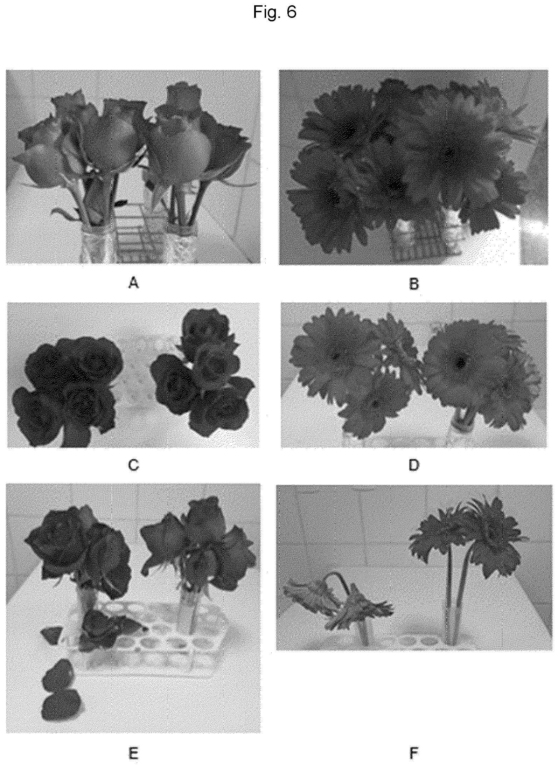

Fig. 6 shows the visual results of an experiment applying a single dose of 100 µM (in water) of an embodiment of the preservative peptide (SEQ ID NO: 15) or water (control) on two types of flowers, all from the same batch and on the same day of acquisition, kept at room temperature (28 °C). Four flowers were used in each container, the stems being kept submerged with at least 5 cm of the respective liquid during the test period. Aluminum foil was used around the container where the stems were submerged, to protect from the incidence of light. Photographic results of flowers kept in each condition (control and test) at 0, 3 and 6 days are shown. In A, C and E are shown images of roses, respectively on days 0, 3 and 6, the left roses kept in H₂O and the right roses in the preservative peptide as indicated above. In B, D and F, images of gerberas are shown, respectively on days 0, 3 and 6, the left gerberas kept in H₂O and the right gerberas in the preservative peptide as indicated above.

### Detailed Description of the Invention

The present invention solves problems of the state of the art by the use of a synthetic peptide for preservation of cells, tissues and/or food and an additive comprising said peptide, being useful to provide improvements in the production, logistics and/or consumption of food and/or plants.

The synthetic peptide in the present invention is selected from the peptide of SEQ ID NO: 1 and SEQ ID NOs: 4 to 14, optionally additionally containing a cell-penetrating peptide tail at the N-terminal portion and/or at the C-terminal portion of the first and/or the last amino acid. The peptide of SEQ ID NO: 3 represents a peptide containing the peptide of SEQ ID NO: 1 and the peptide of SEQ ID NO: 2 in the C-terminal region. The peptide of SEQ ID NO: 15 represents a peptide containing the peptide of SEQ ID NO: 9 and the cell-penetrating peptide of SEQ ID NO: 2.

In one embodiment, the invention provides a topical additive comprising said peptide, for preservation of cells, tissues and/or food and water and/or an excipient.

In one embodiment, the invention provides an additive for use in the hydration liquid of the stem or branch of a flower or inflorescence of ornamental or phytochemical interest.

In one embodiment, the peptide has a cell-penetrating peptide tail at the N-terminal portion and/or the C-terminal portion of the first and/or last amino acid.

The invention is also defined by the following clauses.

Use of the synthetic peptide selected from the peptide of SEQ ID NO: 1 and SEQ ID NOs: 4 to 14, for the preparation of a preservation additive for preserving cells, tissues and/or food.

Use as indicated above wherein said peptide additionally comprises a cell-penetrating peptide tail at the N-terminal portion and/or at the C-terminal portion of the first and/or last amino acid.

Use as indicated above wherein said cell-penetrating peptide tail is of SEQ ID NO: 2.

Use as indicated above wherein said peptide comprises SEQ ID NO: 3.

Use as indicated above wherein said peptide comprises SEQ ID NO: 15.

Use as indicated above for the preparation of a plant and/or fruit preservation additive of agronomic, botanical and/or medicinal interest, or to increase food shelf life and food safety.

Use as indicated above for the preparation of an additive to modulate germination and/or plant development of plants of agronomic, botanical and/or medicinal interest.

Use as indicated above for the preparation of an additive for the treatment of flowers or inflorescences of ornamental or phytochemical interest, to prevent or delay wilting, rot or the occurrence of pests such as bacteria or fungi, and/or to maintain the quality or integrity of the flowers or inflorescences for longer period.

Additive for preservation of cell, tissue and/or food comprising water and/or an excipient and ONE synthetic peptide selected from the peptide of SEQ ID NO: 1 and SEQ ID NOs: 4 to 14.

Additive as indicated wherein said peptide additionally comprises a cell-penetrating peptide tail at the N-terminal portion and/or at the C-terminal portion of the first and/or last amino acid.

Additive as indicated above wherein said cell-penetrating peptide tail is of SEQ ID NO: 2.

Additive as indicated above wherein the synthetic peptide is of SEQ ID NO: 3.

Additive as indicated above wherein said peptide comprises SEQ ID NO: 15.

Additive as indicated above for use in the post-harvest preservation of flowers and/or inflorescences of ornamental or phytochemical interest.

Terms used in this patent application are defined below:
Peptide for incorporation into the intracellular medium. In the context of the present patent application, the term should be understood as any peptide that has the function of helping/allowing a biologically active peptide to be incorporated into the intracellular medium and, thus, presenting the biological effect in the intracellular medium.
Cell cycle. In the context of the present patent application, the cell cycle must be understood as a series of events that occur in cells and that lead to their division and duplication. It should be understood that the cell cycle comprises any state of the cell cycle of microorganisms or plants, such as the quiescence/senescence states regarding interphase and cell division, as well as their phases. Thus, it should be understood that the cell phases G0, G1, S, G2, and mitosis (M) are also part of the cell cycle concept.
Cell-penetrating peptide tail (CPP). In the context of the present patent application, the cell-penetrating peptide tail (CPP) refers to short amino acid sequences that facilitate the entry of the main peptide into the intracellular medium and, in this way, acting as a mode of transport of peptides to the intracellular medium. Still in the context of the present patent application, hydrophilic tails known from the state of the art are included in the CPPs, such as, for example, the tails TAT, SynB1, SynB3, PTD-4, PTD-5, D-Tat, R9-Tat, among others. Also included are amphiphilic tails such as the tails MAP, SBP, FBP and others.
Cell division or multiplication. In the context of the present patent application, cell division or multiplication must be understood as in any type of cell lineage, whether mammalian, plant or microorganism. Still in the context of the present patent application, it should be understood that dividing cells may also be related to the contamination of food or plants with microorganisms and/or the germination of seeds and/or the development of plants.
Inhibition of cell multiplication. In the context of the present patent application, said term should be understood as inhibiting the multiplication of any types or lineages of mammalian, plant or microorganism cells, and the cells may be in any of the stages of cell division, cells may or may not be synchronized in the cell cycle. Inhibiting cell multiplication of cells presents, in the context of the present patent application, the meaning of interrupting the process of cell division, resulting or not in cell death.
Pests or contamination of plants or food. In the context of this patent application, pests or contamination of plants or food are considered to be those biological agents that contaminate plants or food, which are related to their rot and/or that cause damage to the quality or shelf life of their products. Included in this definition are microbial contaminations that lead to rot, those that form toxins and those that alter. Examples include, but are not limited to *P. nicotiana* and P. *idaei, Botrytis cinerea, Colletotrichum spp. Rhizopus stolonifer, S. sclerotiorum, Pestalotia longisetula Rhizoctonia sp., Geotrichum sp., Gnomonia comari, Pilidium concavum* related, among others, to strawberry rot, *Xylella fastidiosa* in coffee and orange groves, fungi associated with the storage, transport and/or medicinal processing of *Cannabis.*
"pep5" or "Pep5". In the context of the present patent application, the term should be understood as the peptide represented by SEQ ID NO: 1, the amino acid sequence being WELVVLGKL.
"cpp". In the context of the present patent application, the term should be understood as being a peptide tail, which can be represented by SEQ ID NO: 2, the amino acid sequence being YGRKKRRQRRR.
pep5-cpp or Pep5-cpp or "pep5-cpp". In the context of the present patent application, the term should be understood as the peptide represented by SEQ ID NO: 1 linked to the peptide tail represented by the sequence SEQ ID NO: 2.
ΔN-pep5-cpp. In the context of the present patent application, the term should be understood as the peptide represented by SEQ ID NO: 1 but without the last amino acid of the N-terminal portion, and linked to the peptide tail represented by the sequence SEQ ID NO: 2.
ΔNi-pep5-cpp. In the context of the present patent application, the term should be understood as the peptide represented by SEQ ID NO: 1 but without the last i amino acids of the N-terminal portion, wherein i = 1, 2, 3, 4 or 5, and linked to the peptide tail represented by the sequence SEQ ID NO: 2.
ΔC-pep5-cpp. In the context of the present patent application, the term should be understood as the peptide represented by SEQ ID NO: 1 but without the last amino acid of the C-terminal portion, and linked to the peptide tail represented by the sequence SEQ ID NO: 2.
ΔCi-pep5-cpp. In the context of the present patent application, the term should be understood as the peptide represented by SEQ ID NO: 1 but without the last i amino acids of the C-terminal portion, wherein i = 1, 2, 3, 4 or 5 and linked to the peptide tail represented by SEQ ID NO: 2. An example is SEQ ID NO: 9.
Peptides from SEQ ID NOs: 4 to 14 are considered within the inventive concept of the present invention due to the small changes in the amino acid sequence in relation to SEQ ID NO: 1 and SEQ ID NO: 9, both effectively tested in the present specification.
Synthesis of peptides. The peptides disclosed by the present patent application were synthesized using the technique of peptide synthesis in solid phase (FastMoc Chemistry, from the company Applied Biosystems).

The examples shown here are intended only to exemplify one of the many ways to carry out the invention, but without limiting its scope.

### Example 1 - Use of the peptide for the preparation of a plant or food preservation additive.

The peptide of SEQ ID NO: 3, which comprises pep5 (SEQ ID NO: 1) and the cell-penetrating peptide (SEQ ID NO: 2), was prepared in a 100 µM solution in water, for direct topical use on the food to be preserved.

The peptide of SEQ ID NO: 15, which comprises the peptide of SEQ ID NO: 9 and the cell penetrating peptide of SEQ ID NO: 2, was prepared in a 100 µM solution in water, for use in the food or plant to be preserved.

Those skilled in the art will immediately understand that they can incorporate other excipients to the additive of the invention in addition to water, to improve the preservation performance shown here.

### Example 2 - Tests of the additive comprising the peptide in the preservation of strawberries against fungal contamination.

The additive prepared according to example 1 was used in experiments for food preservation by single topical application.

Fig. 1 shows the visual results of an experiment involving the application of 100 µM of the preservative peptide (SEQ ID NO: 3) or water (control) on fresh strawberries, all from the same batch and packaging of the commercial product. The results of groups of 10 strawberries in each condition (test and control) at 0, 12, 24, 36, 48 and 60h, respectively in A, B, C, D, E and F are shown.

In D) and E) of Fig. 1, the presence of a fungus with white hyphae is visible, which is not detectable in treated strawberries. Although these contaminants have not been characterized, their appearance is consistent with *Rhizopus stolonifer* or *Sclerotinia sclerotiorum.*

Fig. 2 shows the time in hours (abscissa) and the percentage of spoiled fruits (ordered) after application of 100 µM of preservative peptide (SEQ ID NO: 3) or water on fresh strawberries.

The results in Figs. 1 and 2 clearly show the great potential for fruit preservation through a single application of the additive in aqueous solution.

Similar results are obtained with the application of the peptide of SEQ ID NO: 15, which comprises the peptide of SEQ ID NO: 9 and the peptide of SEQ ID NO: 2.

### Example 3 - Tests of the additive comprising the peptide in the preservation of strawberries against rot.

A new experiment with strawberries, now with the test and control groups being kept at a temperature of 4 °C (in a refrigerator) was conducted to enable the observation of preservation in conditions that do not favor the growth of microorganisms.

Fig. 3 shows the visual results of an experiment applying the additive of the invention with 100 µM of the preservative peptide (SEQ ID NO: 3) in water, or only water as a control on 8 fresh strawberries in each group, all from the same batch and same commercial product packaging. Representative results of strawberries in each condition (test and control) at 0h, 24h, 48h and 5 days, respectively in A, B, C and D are shown.

In D) in Fig. 3, the presence of intense rot in the group of untreated strawberries is very visible, a condition that is not detectable in the group of strawberries treated with the additive of the invention even after 5 days. Said rot is very different from the condition of microbial contamination observed in the experiment of example 2.

Those skilled in the art will immediately understand the great technical and economic value of the present invention and will be able to apply the additive of the invention for the preservation of other plants or foods.

### Example 4 - Tests of the preservative additive comprising the peptide in modulating the germination rate of bean seeds.

To evaluate the effects of the additive of the invention in the modulation of cell division or multiplication in plants, particularly in the modulation of seed germination and/or plant development, an experiment aimed at observing the germination of bean seeds and the subsequent development of seedlings was conducted. Groups of groups of 14 black bean seeds were placed in moist cotton, each group in one condition: control, with application of water; and test, with application of the additive of the invention as described below. The state of the seeds was observed and documented at times 0, 3 and 4, and the pattern and profile of germination of the beans were analyzed.

Fig. 4 shows the visual results of an experiment involving the application of a single dose of 100 µM (in water) of the preservative peptide (Ktut, SEQ ID NO: 3) or water (control) on bean seeds, all from the same batch and same commercial product packaging and kept at room temperature (28 °C). The results of germination of groups of 14 seeds in moist cotton in each condition (control and test) in 0, 3 and 4 days, respectively in A, B and C are shown. The images show that there were differences in the germination pattern of the beans.

Fig. 5 shows the time in days (abscissa) and the number of bean seeds that will germinate (ordered) after application of 100 µM of preservative peptide (Ktut, SEQ ID NO: 3) as indicated in Fig. 4.

The results show substantial modulation of the metabolic state of the shoots or seedlings, as well as the delay in the seed germination process when the mentioned additive is applied. The invention therefore proved to be useful to modulate germination, adjust planting or germination time, among other agronomic or botanical advantages.

Similar results are obtained with the application of the peptide of SEQ ID NO: 15, which comprises the peptide of SEQ ID NO: 9 and the peptide of SEQ ID NO: 2.

### Example 5 - Tests of the additive comprising the preservative peptide in the preservation of post-harvest flowers.

The surprising results obtained in the previous examples motivated the inventor to assess whether the additive of the invention could also provide for the preservation of flowers after their harvest. It is known that flowers and inflorescences are particularly sensitive and tend to wither or rot quickly, which causes technical and economic difficulties in the segments that exploit such plant parts. Special emphasis is placed on the market for ornamental flower and, particularly, on the market for herbal or pharmaceutical products that use flowers or inflorescences as inputs. In these segments, it is particularly desirable to have an approach that preserves the quality and/or integrity of the flowers or inflorescences harvested, so that the subsequent logistical or industrial processing suffers less negative impact.

In this context, as an experimental model to evaluate the preservation of flowers or inflorescences, two different types of flowers and their degradation/preservation profile were evaluated through incubation with water or with a solution of the preservation additive of the invention, keeping the flowers in room temperature for a few days.

The chosen flowers were the rose (*Rose-red*) and the gerbera (*Gerbera*). Species of the genus *Rosa* are among the most ornamental flowers cultivated in the world. Species of the genus *Gerbera* are used as an experimental organism in studies of flowering and flower meristematic development, and because they contain natural coumarin derivatives, they are flowers of phytochemical interest and biological control.

In this embodiment, the peptide of SEQ ID NO: 15, which comprises the peptide of SEQ ID NO: 9 and the peptide of SEQ ID NO: 2, was used.

Fig. 6 shows the visual results of an experiment involving the application of a single dose of 100 µM (in water) of the preservative peptide (SEQ ID NO: 15) or water (control) on two types of flowers, all from the same lot and the same day of acquisition, kept at room temperature (28 °C). Four flowers were used in each container, the stems being kept submerged with at least 5 cm of the respective liquid during the test period. Aluminum foil was used around the container where the stems were submerged, to protect from the incidence of light. Photographic results of flowers kept in each condition (control and test) at 0, 3 and 6 days are shown. In A), C) and E) images of roses are shown, respectively on days 0, 3 and 6, the left roses kept in H₂O and the right roses in the preservative peptide as indicated above. In B), D) and F) images of gerberas are shown, respectively on days 0, 3 and 6, the left gerberas kept in H₂O and the right gerberas in the preservative peptide as indicated above. The images clearly show that there were significant differences in the preservation pattern of the flowers kept in the preservative.

The results in Fig. 6 show the effective preservation of these two different flower species and provide a proof of concept for the use of the additive of the invention both for the segment of flowers and ornamental inflorescences and for the segment of flowers and inflorescences of phytochemical interest, as is the case *Gerbera, Cannabis* and other plant species.

Those skilled in the art will value the knowledge presented herein and will be able to reproduce the invention in the presented embodiments and in other variants, covered in the scope of the appended claims.

## Claims

1. Use of the synthetic peptide selected from the peptide of SEQ ID NO: 1 and SEQ ID NOs: 4 to 14 **characterized in that** it is for the preparation of a preservative additive for the preservation of cells, tissues and/or food, or to increase the time of food shelf and food safety.

2. Use according to claim 1, **characterized in that** said peptide additionally comprises a cell-penetrating peptide tail in the N-terminal portion and/or in the C-terminal portion of the first and/or last amino acid.

3. Use according to claim 2, **characterized in that** said cell-penetrating peptide tail is SEQ ID NO: 2.

4. Use according to claim 3, **characterized in that** said peptide comprises SEQ ID NO: 3 or SEQ ID NO: 15.

5. Use, according to any one of claims 1 to 4, **characterized in that** it is for the preparation of an additive for the preservation of plants and/or fruits of agronomic, botanical and/or medicinal interest.

6. Use according to any one of claims 1 to 4, **characterized in that** it is for the preparation of an additive to modulate the germination and/or plant development of plants of agronomic, botanical and/or medicinal interest.

7. Use according to any one of claims 1 to 4, **characterized in that** it is for the preparation of an additive for the treatment of flowers or inflorescences, to prevent or delay wilting, rot or the occurrence of pests such as bacteria or fungi, and/or maintain the quality or integrity of flowers or inflorescences for a longer period.

8. Additive for the preservation of cells, tissues and/or food **characterized in that** it comprises water and/or an excipient and a synthetic peptide selected from the peptide of SEQ ID NO: 1 and SEQ ID NOs: 4 to 14.

9. Additive according to claim 8, **characterized in that** said peptide additionally comprises a cell-penetrating peptide tail in the N-terminal portion and/or in the C-terminal portion of the first and/or last amino acid.

10. Additive according to claim 9, **characterized in that** said cell-penetrating peptide tail corresponds to SEQ ID NO: 2.

11. Additive according to claim 10, **characterized in that** said peptide comprises SEQ ID NO: 3 or SEQ ID NO: 15.

12. Additive according to claim 8 or 11, **characterized in that** it is for the post-harvest preservation of flowers and/or inflorescences of ornamental or phytochemical interest.
